# EUROPEAN PATENT APPLICATION

(11) **EP 4 321 091 A1**
(43) Date of publication of application: **14.02.2024**
(21) Application number: 23190529.0
(22) Date of filing: 09.08.2023
(51) Int. Cl.: A61B 5/107, A61B 5/287, A61B 5/00, A61B 18/14, A61B 34/20, A61B 18/00

(54) **VISUALIZING AND CLUSTERING MULTIPLE ELECTRODES OF A HIGH-DEFINITION CATHETER PROJECTED ON TISSUE SURFACE**

(30) Priority: 10.08.2022 US 202217885407
(71) Applicant: BIOSENSE WEBSTER (ISRAEL) LTD., 2066717 Yokneam (IL)
(72) Inventor: MASSARWA, Fady, 2066717 Yokneam (IL); DERECH, Niv, 2066717 Yokneam (IL); COHEN, Assaf, 2066717 Yokneam (IL); SHEINER, Amiran, 2066717 Yokneam (IL); SHTIRBERG, Illya, 2066717 Yokneam (IL); SCHECHTER, Menachem, 2066717 Yokneam (IL)
(74) Representative: Carpmaels & Ransford LLP

(57) **Abstract**

A system includes a display and a processor. The processor is configured to: (i) estimate, based on signals, multiple regions of tissue on a surface of an organ, which are affected by multiple respective electrodes positioned in the organ, (ii) calculate, for at least a subset of the electrodes, a unified region of the tissue affected by the subset, and (iii) display a mark indicative of the unified region on the display.

## Description

### FIELD OF THE DISCLOSURE

The present disclosure relates generally to medical devices, and particularly to methods and systems for visualizing multiple electrodes of a high-definition catheter projected on tissue.

### BACKGROUND OF THE DISCLOSURE

Various techniques for visualizing catheters and tissue in question have been published.

For example, U.S. Patent 10,376,320 describes a three-dimensional surface representation of the anatomic structure constrained relative to one or more anchor portions corresponding to received input regarding the location of anatomic features of the anatomic structure.

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based tracking and ablation system, in accordance with an example of the present disclosure;
Fig. 2 is a schematic, pictorial illustration of multi-electrode catheter and electrodes thereof projected on tissue surface, in accordance with an example of the present disclosure;
Fig. 3 is a schematic, pictorial illustration of catheter electrodes clustered and projected on tissue surface, in accordance with another example of the present disclosure; and
Fig. 4 is a flow charts that schematically illustrate a method for visualizing the distance between a cluster of multiple electrodes and the surface of tissue in patient heart, in accordance with examples of the present disclosure.

### DETAILED DESCRIPTION OF EXAMPLES

### OVERVIEW

Some medical procedures, such as electrophysiology (EP), require the insertion of one or more multi-electrode catheters of an EP system into a patient heart, and using the electrodes for diagnosing and/or treating arrhythmia in the heart. In some cases, it is important to display to a user of the system (e.g., a physician), the distance between the electrode(s) and the surface of heart tissue. Some of the catheters, however, have a large number of electrodes (e.g., about 48) that are grouped in close proximity to one another, and therefore, displaying the distance of each electrode from the surface may have too many marks that may confuse the user and interfere with the procedure.

Examples of the present disclosure that are described hereafter provide improved techniques for displaying, to a user, various parameters related to a multi-electrode catheter, such as but not limited to the distance between multiple electrodes and tissue during an EP procedure.

In some examples, a system for sensing and treating arrhythmia comprises a high-definition catheter having multiple (e.g., about 48) adjacent electrodes. In the context of the present disclosure, the term "high-definition catheter" refers to a catheter having multiple branches, each branch comprising multiple electrodes. For example, an OPTRELL^{™} catheter, which is produced by Biosense Webster Inc. (Irvine, Calif.), and is configured for mapping arrhythmia in the patient heart using multiple sensing electrodes thereof. The catheter comprises one or more position sensors configured to produce signals indicative of the catheter position in a predefined XYZ coordinate system.

In some examples, the system comprises a display and a processor, which is configured to receive the position signals and other signals from the catheter. The processor is configured to hold information comprising the distance of at least one of, and typically, each electrode from the position sensor of the catheter. In some examples, based on the information and the signals received from the catheter, the processor is configured to estimate the distance of each electrode from the closest surface of the heart. The processor is further configured to cluster the electrodes into multiple groups of adjacent electrodes, and to display over an anatomical map of the heart, marks indicative of one or both of: (i) a projection of each electrode on the heart surface and an indication of the distance of each electrode from the closest surface, and (ii) groups of adjacent electrodes located at a similar distance from the closest surface.

In an example, the catheter may comprise first and second splines whose electrodes are positioned at first and second respective distances, which are different from one another, from the closest heart surface. In this example, the processor is configured to calculate the first and second distances, and to display over the closest surface of the anatomical (or electro-anatomical) map of the heart, first and second respective marks that are indicative of the first and second distances. In the example of the OPTRELL^{™} catheter, the processor is configured to display a polygon surrounding the region covered by the electrodes of the catheter distal end. The outline of the polygon comprises the first and second marks shown as first and second lines, respectively. In the present example, the first distance is smaller than the second distance, and therefore, the first line is thicker than the second line, and the thickness of each line is indicative of the distance of the respective spline electrodes from the closest surface of the heart.

In other examples, the first and second lines of the polygon may differ from one another in the color, shape, size, or any combination thereof. Moreover, in addition to or instead of a polygon, the processor is configured to display any suitable type of the first mark and second mark indicative of the first and second distances of the first and second groups of electrodes, respectively, from the closest surface of the heart.

The disclosed techniques improve the presentation quality of data related to high-definition catheters having multiple (e.g., tens of) adjacent electrodes displayed to a user during medical procedures.

### SYSTEM DESCRIPTION

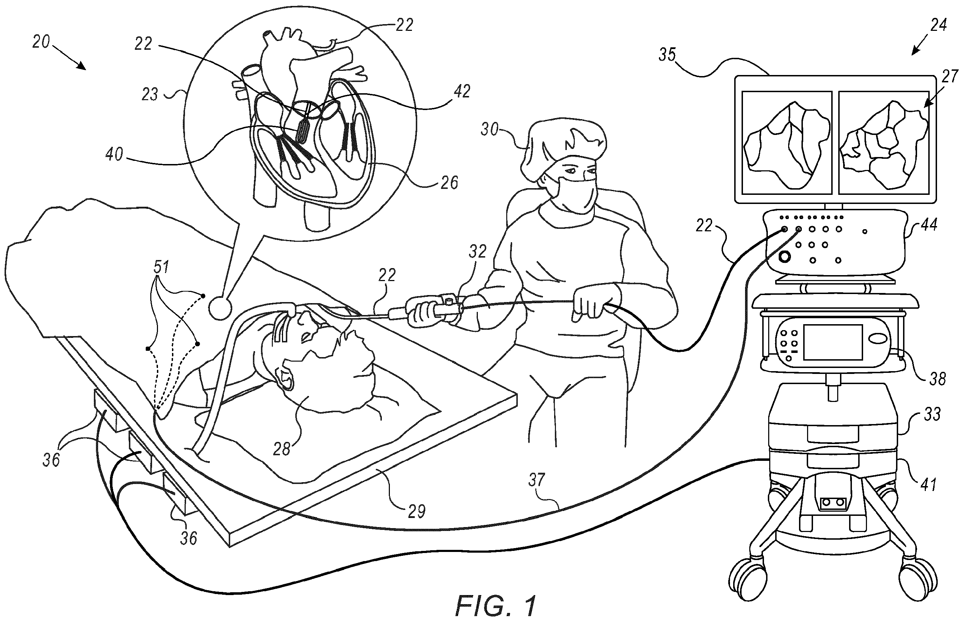

Fig. 1 is a schematic, pictorial illustration of a catheter-based tracking and ablation system 20, in accordance with an example of the present disclosure.

In some examples, system 20 comprises a catheter 22, which is configured to carry out cardiac procedures, and a control console 24. In the example described herein, catheter 22 may be used for any suitable therapeutic and/or diagnostic purposes, such as sensing electro-anatomical signals and/or ablation of tissue in a heart 26. In the context of the present disclosure and in the claims, the term "ablation" refers to a radiofrequency (RF) ablation procedure or to an irreversible electroporation (IRE) procedure, which is different from the RF ablation, but the difference between the RF ablation and the IRE is not affecting the essence of the present disclosure. The structure and functionality of catheter 22 is described in detail hereinafter.

In some examples, console 24 comprises a processor 33, typically a general-purpose computer, with suitable front end and interface circuits for receiving signals via catheters 22 and for controlling the other components of system 20 described herein. Console 24 further comprises a user display 35, which is configured to receive from processor 33 graphical and/or textual display items, such as a map 27 of heart 26, and to display map 27.

In some examples, map 27 may comprise any suitable type of three-dimensional (3D) anatomical map produced using any suitable technique. For example, the anatomical map may be produced using an anatomical image produced by using a suitable medical imaging system, or using a fast anatomical mapping (FAM) technique available in the CARTO^{™} system, produced by Biosense Webster Inc. (Irvine, Calif.), or using any other suitable technique, or using any suitable combination of the above.

In some examples, console 24 comprises a recording unit 38, which is configured to record in case of failure in the CARTO^{™} and/or a failure to pace in certain electrodes, a patient interface unit (PIU) 44, which is configured to exchange signals between console 24 and multiple entities (e.g., catheter 22) of system 20.

Reference is now made to an inset 23. In some examples, prior to performing an ablation procedure, a physician 30 inserts one or more catheters, such as catheter 22, through the vasculature system of a patient 28 lying on a table 29, so as to perform electro-anatomical (EA) mapping of tissue in question of heart 26.

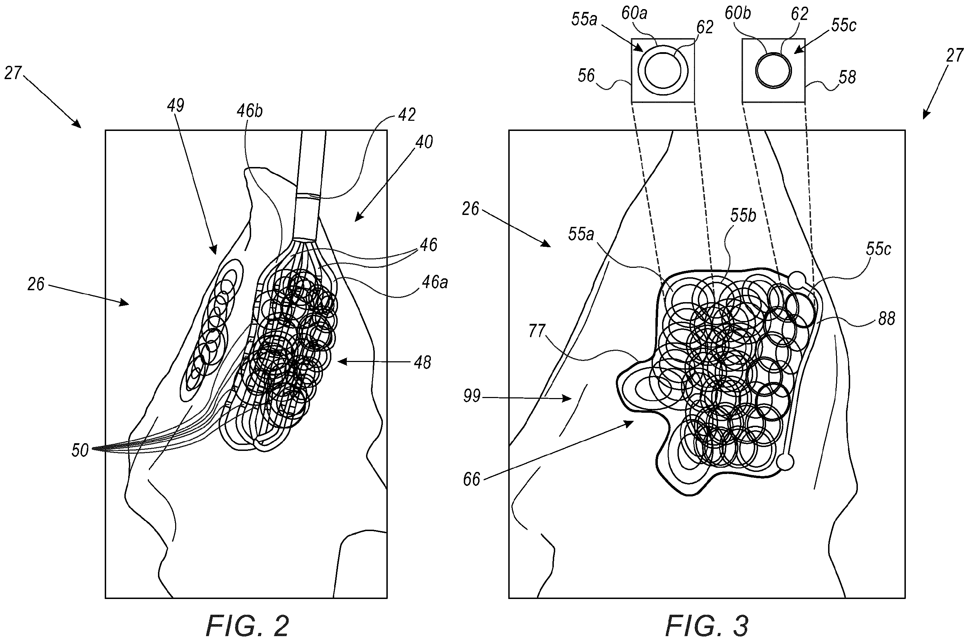

In some examples, catheter 22 comprises a distal-end assembly or end effector 40, in the present example, an OPTRELL^{™} catheter (shown in Fig. 2 below), which is produced by Biosense Webster Inc. (Irvine, Calif.) and is configured for mapping arrhythmia using multiple sensing electrodes thereof. Various components of the catheter of Fig. 2 are shown and described in U.S. Patent Application Publication No. US-2020-0345262-A1, which is incorporated by reference as if set forth in full herein to this application. Each sensing electrode is configured to produce, in response to sensing electrophysiological (EP) signals in tissue of heart 26, one or more signals indicative of the sensed EP signals.

In other examples, distal-end assembly or catheter end effector 40 may comprise: (i) a basket catheter having multiple splines, each spline having multiple sensing electrodes, (ii) a balloon catheter having multiple sensing electrodes disposed on the surface of the balloon, or (iii) a focal catheter having multiple sensing electrodes. It is noted that the sensing electrodes (which receives signals from tissue) can operate to ablate tissues (by sending electrical signals (RF or IRE) into tissues).

In some examples, the proximal end of catheter 22 is connected, *inter alia,* to interface circuits (not shown) of PIU 44, to transfer these signals from the sensing or ablating electrodes to processor 33 for performing the EA mapping.

In the context of the present disclosure and in the claims, the terms "about" or "approximately" for any numerical values or ranges indicate a suitable dimensional tolerance that allows the part or collection of components to function for its intended purpose as described herein.

In other examples, catheter 22 may comprise one or more ablation electrodes (not shown) coupled to distal-end assembly 40. The ablation electrodes are configured to ablate tissue at a target location of heart 26, which is determined based on the analysis of the EA mapping of the tissue in question of heart 26. After determining the ablation plan, physician 30 navigates distal-end assembly 40 in close proximity to the target location in heart 26 e.g., using a manipulator 32 for manipulating catheter 22. Subsequently, physician 30 places one or more of the ablation electrodes (of a selected catheter) in contact with the target tissue, and applies, to the tissue, one or more ablation signals. Additionally, or alternatively, physician 30 may use any different sorts of suitable catheters for ablating tissue of heart 26 in order to carry out the aforementioned ablation plan. It is noted that the ablation electrodes can be separate from the mapping or sensing electrodes in some embodiments. In such cases, the separate ablation electrodes can be used to sense tissue signals but with sacrifices in signal to noise or resolution in such sensing capability.

In some examples, the position of distal-end assembly 40 in the heart cavity is measured using a position sensor 42 of a magnetic position tracking system. The position sensor 42 can be a magnetic position sensor such as described with reference to element 42 in Fig. 3 of U.S. Patent Application Publication No. US-2020-0345262-A1. In the present example, console 24 comprises a driver circuit 41, which is configured to drive magnetic field generators 36 placed at known positions external to patient 28 lying on table 29, e.g., below the patient's torso. Position sensor 42 is coupled to the distal end, and is configured to generate position signals in response to sensed external magnetic fields from field generators 36. The position signals are indicative of the position the distal end of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

In some examples, the coordinate system of the position tracking system is registered with the coordinate systems of system 20 and map 27, so that processor 33 is configured to display, the position of distal-end assembly 40, over the anatomical or EA map (e.g., map 27).

In some examples, system 20 comprises external patch electrodes 51 coupled to the skin of the chest of patient 28, and typically one or more additional indifferent electrodes (not shown) coupled to the skin of the back of patient 28. Electrodes 51 are configured to sense signals indicative of impedance measured between electrodes of distal-end assembly 40 (shown in detail in Fig. 2 below) and electrodes 51.

In some examples, based on the signals received from the electrodes of DEA 40 and external patch electrodes 51, processor 33 is configured to produce additional position signals, indicative of the position of each of the electrode of DEA 40 in the XYZ coordinate system described above. The position signals are produced using an Advanced Catheter Location (ACL) catheter-position tracking method. In the present example, processor 33 is connected to patch electrodes 51 via electrical wires running through a cable 37 and PIU 44.

In some examples, processor 33 is configured to determine the position coordinates of each electrode of DEA 40, based on impedances measured between each electrode of DEA 40 and each of patch electrodes 51. The ACL method of electrode position sensing using system 20 is implemented in various medical applications, for example in the CARTO^{™} system, produced by Biosense Webster Inc. (Irvine, California) and is described in more detail, for example, in U.S. Patents 7,756,576, 7,869,865, and 7,848,787.

In some examples, processor 33, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems.

### PROJECTING ELECTRODES OF CATHETER ON TISSUE SURFACE

Fig. 2 is a schematic, pictorial illustration of distal-end assembly 40 having multiple electrodes 50, and visualization, over map 27, of electrodes 50 projected on tissue surface of heart 26, in accordance with an example of the present disclosure.

In some examples, distal-end assembly 40 comprises multiple (e.g., about six) splines 46, 46a and 46b, and multiple electrodes coupled along each of the splines, which are positioned in heart 26. In an example, the configuration of distal-end assembly 40 (e.g., about 48 electrodes 50) enables high resolution mapping (and/or ablation) of the respective section of tissue of heart 26.

In some examples, processor 33 is configured to estimate, based on signals received, e.g., from the position tracking system, the distance between each electrode 50 and the surface of heart 26. More specifically, processor 33 holds information comprising the distance of each electrode from position sensor 42, and based on: (i) the position signal, (ii) the anatomical mapping of the tissue, and (iii) the distance between position sensor 42 and a given electrode 50. Based on the (i) position signals received from position sensor 42 and from the ACL system described in Fig. 1 above, (ii) anatomical map 26, and (iii) distance between position sensor 42 and a given electrode 50, processor 33 is configured to estimate the distance between given electrode 50 and the geometrical mapping of heart 26.

In the context of the present disclosure, the term geometrical mapping refers to a three-dimensional (3D) mapping of the surface of heart 26. In the present example, the section of the heart surface located in close proximity or in contact with the respective electrodes 50.

Note that in the example of DEA 40, splines 46, 46a and 46b are flexible, and therefore, the distance between each electrode 50 and the closest surface of heart 26 may differ among electrodes 50. For example, in case spline 46a is bending toward the surface of heart 26 more than spline 46b, the respective electrodes 50 of spline 46a are expected to be closer to the surface of heart 26 compared to electrodes 50 of spline 46b. In some examples, processor 33 can estimate the position of DEA 40 based on the position signals received from position sensor 42. However, in addition to that of position sensor 42, based on the signals from the ACL system that are indicative of the measured impedance between each electrode 50 and patch electrodes 51, processor 33 is configured to estimate the position of each of the approximately 48 electrodes 50 in the XYZ coordinate system. Based on the geometrical mapping of heart 26, and the position of each electrode 50 in the XYZ coordinate system, processor 33 is configured to estimate the distance between each electrode 50 and the closest surface of heart 26.

In some examples, processor 33 is configured to display, e.g., over map 27, a mark indicative of the distance between each electrode 50 and the surface of heart 26. In the example of Fig. 2, processor 33 is configured to display an array 49 of multiple marks indicative the projection of electrodes 50 of spline 46b on the surface of the tissue of heart 26. In the present example, the marks can be in the form of circles with varying diameters whereby the smaller diameter indicates that the electrode at the center of the circle is close to the heart tissue and a wider diameter indicates that the electrode is further from the heart tissue. Examples of such marks are described in more detail in Fig. 3 below.

Additionally, or alternatively, based on at least the signals described above, processor 33 is configured to display, over map 27, multiple regions of the tissue on the surface of heart 26, which are affected by respective electrodes 50 of distal-end assembly 40. For example, array 49 (or any other array of marks) representing regions affected by respective electrodes may be indicative of the contact force and the amount of energy intended to be applied, by each electrode 50, to a respective region one the tissue surface of heart 26.

In the present example, among the splines of distal-end assembly 40, spline 46a is placed in the closest proximity to the surface of the tissue of heart 26. Processor 33 is configured to display arrays 48 of marks that are indicative the respective electrode 50 of splines 46, and 46a the are projected on the surface of heart 26.

### CLUSTERING ELECTRODES OF CATHETER PROJECTED ON TISSUE SURFACE

Fig. 3 is a schematic, pictorial illustration of the projection and clustering of electrodes 50 over map 27, in accordance with another example of the present disclosure.

In some examples, processor 33 is configured to display, over map 27, the projection of electrodes 50 of all the splines of distal-end assembly 40 (shown in Fig. 2 above). In the example of Fig. 3, processor 33 displays an array 66 of marks 55, which are indicative of the proximity of the respective electrodes 50 to the surface of the tissue of heart 26. More specifically, marks 55a, 55b and 55c are indicative of the distance of respective electrodes 50 from the tissue surface.

In some examples, due to the large number (e.g., about 48) of electrodes 50, some marks 55 (e.g., marks 55a and 55b) of array 66 may overlap one another. The overlap shown in Fig. 2 may create a visual noise that may confuse physician 30 and other users of system 20.

Reference is now made to insets 56 and 58 showing marks 55a and 55c, respectively. In some examples, mark 55a comprises an outer circle 60 and an inner circle 62a, and mark 55b comprise outer circle 60 and an inner circle 62b.

In an example, inner circle 62 is indicative of the position of the respective electrode 50 and the center of the projection thereof on the surface of heart 26. Therefore, inner circle 62 typically has a constant size for all marks 55. Outer circles 60a and 60b are indicative of respective regions on the surface of heart 26 that are affected by the respective electrodes 50.

In the context of the present disclosure and in the claims, the term "affected" refers to one or more parameters of the medical procedure that are performed using distal-end assembly 40, and are related to the respective tissue of heart 26. As used herein, the term "affected by respective electrodes" means that the respective electrodes 50 are in contact with or in close proximity with heart tissue for recording of ECG or for delivering energy to the tissue. For example, the size (e.g., diameter) of outer circles 60a and 60b is indicative of the proximity between a given electrode 50 and the tissue surface of heart 26 in which the heart tissue is "affected" by the given electrode 50. In the present example, the electrode whose projection is shown by mark 55c appears to be closer to the surface of heart 26 compared to the electrode whose projection is shown by mark 55a. As described in Fig. 2 above, among the splines of distal-end assembly 40, spline 46a is placed in the closest proximity to the surface of the tissue of heart 26. In such examples, processor 33 is configured to display circle 60a having the same diameter of circle 62 in mark 55c, whereas in mark 55b, circle 60b has a larger diameter compared to that of circle 62. In more general terms, the diameter difference between the inner circle 62 and outer circle 60 of a given mark 55, is indicative of the distance between the respective electrode 50 and the surface of heart 26.

In other examples, mark 55 may comprise any other suitable shape, such as an ellipse, a rectangle, or a square, instead of or in addition to circles 60 and 62. For example, mark 55 may comprise a circle and a square, or the circles may have a different thickness, and/or a different color, and/or a different texture (e.g., solid line and broken line).

In alternative examples, the outer circle 60 of mark 55 may appear larger when the respective electrode 50 is closer to the surface of heart 26.

Reference is now made back to the general view of Fig. 2 above. In the example of Fig. 2, marks 55 of array 49 appear to be separated from marks 55 of array 48, however, both arrays are projections of marks 55 of distal-end assembly 40. Reference is now made back to the general view of Fig. 3. In some examples, processor 33 is configured to cluster all marks 55 of distal-end assembly 40 in a single array (e.g., array 66). However, the proximity (e.g., less than about 3 mm in OPTRELL^{™} catheter) between adjacent electrodes 50 may cause overlap between two or more adjacent marks 55. As shown in Fig. 3, the overlapping and mixing of the circles of marks 55 may confuse physician 30, and therefore, may cause a wrong interpretation of marks 55.

In some examples, processor 33 is configured to calculate, for at least a subset of array 66, first and second clusters of marks 55 indicative of respective first and second arrays electrodes 50 that are positioned at first and second different distances, respectively, from the surface of heart 26. In the example of Fig. 3, processor 33 is configured to display a polygon 99 surrounding marks 55 indicative of the projection and proximity level of respective electrodes 50 to the surface of heart 26.

In the present example, polygon 99 comprises lines 77 and 88, each of which representing a different section of array 66. Line 88 is indicative of marks 55 whose respective electrodes 50 are closer to the surface of heart 26, compared to the electrodes 50 represented by line 77. In the example of Fig. 3, line 88 appears thicker than line 77. The higher thickness is indicative of closer proximity, of the respective cluster of electrodes 50, to the surface of heart 26. For example, mark 55c is indicative of a first electrode 50 positioned closer to the surface of heart 26 compared to a second electrode represented by mark 55a. Therefore, processor 33 is configured to display (thicker) line 88 near mark 55c and (thinner) line 77 near marks 55a and 55b, and may also display an additional line, e.g., an island-shaped or peninsula-shaped line (not shown), within the region surrounded by polygon 99. The island or peninsula may be indicative of an additional cluster of electrodes 50 located at a distance from the surface of heart 26, which is different from that of electrodes 50 near lines 77 and 88. Note that physician 30 (or any other user of system 20) may select whether he or she wants processor 33 to display: (i) both marks 55 and the lines of polygon 99 at the same time (as shown in Fig. 3), (ii) only one or both lines 77 and 88, or (iii) only some or all of marks 55. Moreover, the user of system 20 may toggle between the above options during the medical procedure.

In other examples, instead of or in addition to the thickness difference between lines 77 and 88 (and/or other marks) of polygon 99, processor 33 is configured to display any other suitable lines or marks indicative of the proximity between respective electrodes 50 and the surface of heart 26. For example, (i) dashed lines and solid lines, (ii) different color of lines, and (iii) any suitable combination thereof.

In other examples, marks 55, lines 77 and 88 and other types of marks may be used to visualize regions of the tissue of heart 26 that are affected by electrodes 50. For example, when sensing electrocardiogram (ECG) signals in the tissue, the distance between a given electrode 50 and the tissue affects the properties of the signals produced by the given electrode 50 responsively to sensing the ECG signal in heart 26. Moreover, in case electrodes 50 are configured to apply ablation pulses to the tissue, the properties of the lesion formed in the ablated tissue depend on various parameters that can be visualized by the aforementioned marks and lines. For example, the electrode-tissue contact force and the amount of energy (e.g., power and duration of ablation pulses) applied to the tissue may affect the size of the formed lesion. In this example, the thickness and/or color and/or texture of the lines of polygon 99 may be indicative of the lesion size at the respective segments or sections within the region surrounded by polygon 99. In other words, processor 33 is configured to calculate, for at least a subset of electrodes 50 (and typically to different sections within the area surrounded by polygon 99), a unified region of the tissue of heart 26 that is affected by the subset of electrodes 50. In the context of the present disclosure and in the claims, the term "unified region" refers to an outer perimeter curve that bounds the region affected by a plurality of respective electrodes 50. In the example of lines 77 and 88, line 88 is indicative of marks 55 whose respective electrodes 50 are closer to the surface of heart 26, compared to the electrodes 50 represented by line 77. Moreover, processor 33 is configured to display the marks and/or lines over map 27 to visualize a quantitative metric of effects, of the operations carried out using the respective one or more subsets of electrodes 50, on the tissue of heart 26.

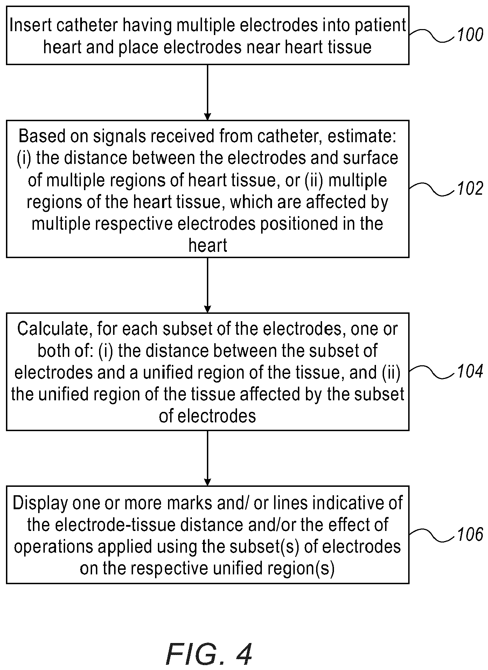

Fig. 4 is a flow charts that schematically illustrate a method for visualizing the distance between a cluster of multiple electrodes and the surface of tissue in heart 26, in accordance with examples of the present disclosure.

The method begins at a catheter insertion step 100, with physician 30 inserting distal-end assembly 40 (of catheter 22) and electrodes 50 thereof into heart 26, and placing electrodes 50 near the surface of heart 26, as described in detail in Figs. 1-3 above.

At a signal receiving and estimation step 102, processor 33 receives signals from catheter 22, such as but not limited to signals indicative of the position of one or more of electrodes 50, the position may be absolute position (e.g., in the XYZ coordinate system of the position tracking system described in Fig. 1 above) or the position of electrodes 50 relative to the surface of heart 26, as described in Figs. 1 and 2 above. In some examples, based on the received signals, processor 33 is configured to estimate, the distance between one or more subsets of electrodes 50 and one or more respective regions of tissue on the surface of heart 26.

In other examples, in an ablation procedure, the signals may be indicative of at least one of: (i) contact force between electrodes 50 and the respective regions of the tissue on the surface of heart 26, (ii) energy (e.g., power and duration) of ablation pulses intended to be applied to the tissue, and (iii) any other parameters related to the ablation procedure. In such examples, based on the signals, processor 33 is configured to estimate multiple regions of the heart tissue, which are affected by one or more respective subsets of electrodes 50 that are positioned in heart 26.

At a calculation step 104, processor 33 is configured to calculate, for each subset of electrodes 50, the distance between the subset of electrodes 50 and a unified region of the tissue, as described in detail in Fig. 3 above. Additionally, or alternatively, processor 33 is configured to calculate the unified region of the tissue of heart 26 that is affected by the subset of electrodes 50.

At a displaying step 106 that concludes the method, based on the calculation of step 104 above, processor 33 is configured to display, e.g., on display 35, one or more marks (e.g., marks 55) and/or lines (e.g., lines 77 and 88), which are indicative of one or both: (i) the distance between electrodes 50 of the subsets and the tissue surface of the respective regions of heart 26, and (ii) the effect of the operations carried out using the subsets of electrodes 50 on the respective unified region(s) of the tissue of heart 26.

Although the examples described herein mainly address electrophysiology procedures carried out in patient heart, the methods and systems described herein can also be used in other applications, such as in any catheterization procedure using multi-electrode catheters or probes inserted into the heart or any other target organ of a patient.

### Example 1

A system (20) including a display (35) with a catheter end effector (40) having a plurality of electrodes to sense signals, and a processor (33) connected to the display and electrodes of the end effector, which is configured to: (i) estimate, based on signals, multiple regions (55a, 55b, 55c) of tissue on a surface of an organ (26), which are affected by multiple respective electrodes (50) positioned in the organ (26), (ii) calculate, for at least a subset of the electrodes (50), a unified region (99) of the tissue affected by the subset, and (iii) display a mark (77, 88) indicative of the unified region (99) on the display (35).

### Example 2

The system according to Example 1, wherein the signals include position signals indicative of respective positions of the multiple respective electrodes, and wherein the processor is configured to calculate one or more distances between a surface of the tissue and one or more of the electrodes of the subset, respectively.

### Example 3

The system according to Example 2, wherein the processor is configured to display the mark, which is indicative of the distance between the unified region and the subset of the electrodes.

### Example 4

The system according to Example 3, wherein the position signals include first and second position signals indicative of first and second positions of first and second subsets of the electrodes, respectively, and wherein, based on the first and second position signals, the processor is configured to calculate: (i) a first distance between the first subset and a first surface of a first unified region, and (ii) a second distance between the second subset and a second surface of a second unified region, different from the first surface of the first unified region.

### Example 5

The system according to Example 4, wherein the first distance is different from the second distance, and wherein the processor is configured to display: (i) over a first map of the first unified region, a first mark, which is indicative of the first distance, and (ii) over a second map of the second unified region, a second mark, which is different from the first mark and is indicative of the second distance.

### Example 6

The system according to Example 5, wherein the processor is configured to display a polygon surrounding at least the first map and the second map, and wherein an outline of the polygon includes the first and second marks.

### Example 7

The system according to Example 6, wherein the first mark includes a first line, which is a first section of the outline of the polygon, and the second mark includes a second line, which is a second section of the outline of the polygon.

### Example 8

The system according to Example 7, wherein the first line has a first color, and the second line has a second color, different from the first color.

### Example 9

The system according to Example 7, wherein the first line includes a solid line, and the second line includes a broken line.

### Example 10

The system according to Examples 1-9, wherein the signals are indicative of one or more parameters of an ablation of the tissue, and wherein the processor is configured to: (i) calculate, based on the signals, a size of a lesion intended to be formed by the subset in the unified region, and (ii) display, over the uniform region, the mark, which is indicative of the size of the lesion.

### Example 11

A method, including:
estimating, based on signals, multiple regions (55a, 55b, 55c) of tissue on a surface of an organ (26), which are affected by multiple respective electrodes (50) positioned in the organ (26);
calculating, for at least a subset of the electrodes (50), a unified region (99) of the tissue affected by the subset; and
displaying a mark (77, 88) indicative of the unified region (99).

### Example 12

The method according to Example 11, wherein the signals include position signals indicative of respective positions of the multiple respective electrodes, and wherein calculating the unified region includes calculating one or more distances between a surface of the tissue and one or more of the electrodes of the subset, respectively.

### Example 13

The method according to Example 12, wherein the mark is indicative of the distance between the unified region and the subset of the electrodes.

### Example 14

The method according to Example 13, wherein the position signals include first and second position signals indicative of first and second positions of first and second subsets of the electrodes, respectively, and wherein, based on the first and second position signals, calculating the unified region includes calculating: (i) a first distance between the first subset and a first surface of a first unified region, and (ii) a second distance between the second subset and a second surface of a second unified region, different from the first surface of the first unified region.

### Example 15

The method according to Example 14, wherein the first distance is different from the second distance, and wherein displaying the mark includes displaying: (i) over a first map of the first unified region, a first mark, which is indicative of the first distance, and (ii) over a second map of the second unified region, a second mark, which is different from the first mark and is indicative of the second distance.

### Example 16

The method according to Example 15, wherein displaying the first and second marks includes displaying a polygon surrounding at least the first map and the second map, and wherein an outline of the polygon includes the first and second marks.

### Example 17

The method according to Example 16, wherein the first mark includes a first line, which is a first section of the outline of the polygon, and the second mark includes a second line, which is a second section of the outline of the polygon.

### Example 18

The method according to Example 17, wherein the first line has a first color, and the second line has a second color, different from the first color.

### Example 19

The method according to Example 17, wherein the first line includes a solid line, and the second line includes a broken line.

### Example 20

The method according to Examples 11-19, wherein the signals are indicative of one or more parameters of an ablation of the tissue, and wherein: (i) calculating the unified region includes calculating, based on the signals, a size of a lesion intended to be formed by the subset in the unified region, and (ii) displaying the mark includes displaying, over the uniform region, the mark, which is indicative of the size of the lesion.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A system (20), comprising:
a display (35); and
a catheter end effector (40) having a plurality of electrodes disposed thereon to sense signals;
a processor (33) electrically connected to the display and the plurality of electrodes, which is configured to
estimate, based on the signals, multiple regions (55a, 55b, 55c) of tissue on a surface of an organ (26), which are affected by multiple respective electrodes (50) positioned in the organ (26);
calculate, for at least a subset of the electrodes (50), a unified region (99) of the tissue affected by the subset; and
display a mark (77, 88) indicative of the unified region (99) on the display (35).

2. The system according to claim 1, wherein the signals comprise position signals indicative of respective positions of the multiple respective electrodes, and wherein the processor is configured to calculate one or more distances between a surface of the tissue and one or more of the electrodes of the subset, respectively.

3. The system according to claim 2, wherein the processor is configured to display the mark, which is indicative of the distance between the unified region and the subset of the electrodes.

4. The system according to claim 3, wherein the position signals comprise first and second position signals indicative of first and second positions of first and second subsets of the electrodes, respectively, and wherein, based on the first and second position signals, the processor is configured to calculate: (i) a first distance between the first subset and a first surface of a first unified region, and (ii) a second distance between the second subset and a second surface of a second unified region, different from the first surface of the first unified region.

5. The system according to claim 4, wherein the first distance is different from the second distance, and wherein the processor is configured to display: (i) over a first map of the first unified region, a first mark, which is indicative of the first distance, and (ii) over a second map of the second unified region, a second mark, which is different from the first mark and is indicative of the second distance.

6. The system according to claim 5, wherein the processor is configured to display a polygon surrounding at least the first map and the second map, and wherein an outline of the polygon comprises the first and second marks.

7. A method, comprising:
estimating, based on signals from a plurality of electrodes, multiple regions (55a, 55b, 55c) of tissue on a surface of an organ (26), which are affected by multiple respective electrodes (50) positioned in the organ (26);
calculating, for at least a subset of the plurality of electrodes (50), a unified region (99) of the tissue affected by the subset; and
displaying a mark (77, 88) indicative of the unified region (99).

8. The method according to claim 7, wherein the signals comprise position signals indicative of respective positions of the multiple respective electrodes, and wherein calculating the unified region comprises calculating one or more distances between a surface of the tissue and one or more of the electrodes of the subset, respectively.

9. The method according to claim 8, wherein the mark is indicative of the distance between the unified region and the subset of the electrodes.

10. The method according to claim 9, wherein the position signals comprise first and second position signals indicative of first and second positions of first and second subsets of the electrodes, respectively, and wherein, based on the first and second position signals, calculating the unified region comprises calculating: (i) a first distance between the first subset and a first surface of a first unified region, and (ii) a second distance between the second subset and a second surface of a second unified region, different from the first surface of the first unified region.

11. The method according to claim 10, wherein the first distance is different from the second distance, and wherein displaying the mark comprises displaying: (i) over a first map of the first unified region, a first mark, which is indicative of the first distance, and (ii) over a second map of the second unified region, a second mark, which is different from the first mark and is indicative of the second distance.

12. The method according to claim 11, wherein displaying the first and second marks comprises displaying a polygon surrounding at least the first map and the second map, and wherein an outline of the polygon comprises the first and second marks.

13. The system according to claim 6 or the method according to claim 12, wherein the first mark comprises a first line, which is a first section of the outline of the polygon, and the second mark comprises a second line, which is a second section of the outline of the polygon.

14. The system or the method according to claim 13, wherein the first line has a first color, and the second line has a second color, different from the first color.

15. The system or the method according to claim 13, wherein the first line comprises a solid line, and the second line comprises a broken line.

16. The method according to any of claims 7 to 15, wherein the signals are indicative of one or more parameters of an ablation of the tissue, and wherein: (i) calculating the unified region comprises calculating, based on the signals, a size of a lesion intended to be formed by the subset in the unified region, and (ii) displaying the mark comprises displaying, over the uniform region, the mark, which is indicative of the size of the lesion.

17. The system according to any of claims 1 to 6 and 13 to 15, wherein the signals are indicative of one or more parameters of an ablation of the tissue, and wherein the processor is configured to: (i) calculate, based on the signals, a size of a lesion intended to be formed by the subset in the unified region, and (ii) display, over the uniform region, the mark, which is indicative of the size of the lesion.
